# EUROPEAN PATENT APPLICATION

(11) **EP 2 022 535 A1**
(43) Date of publication of application: **11.02.2009**
(21) Application number: 07015545.2
(22) Date of filing: 07.08.2007
(51) Int. Cl.: A61Q 5/04, A61K 8/58, A61K 8/891

(54) **Composition for permanent shaping human hair**

(71) Applicant: KPSS-Kao Professional Salon Services GmbH, 64297 Darmstadt (DE)
(72) Inventor: Hoffmann, Martin, 64673 Zwingenberg (DE); Molenda, Michael, 60487 Frankfurt (DE); Grit, Mustafa, Dr., 64579 Gernsheim (DE)

(57) **Abstract**

The present invention concerns a composition for the permanent shaping of human hair used both for the permanent waving of human hair with an excellent waving effect as well as for the straightening of curled hair comprising at least one arylated silicone, preferably trimethyl pentaphenyl trisiloxane.

## Description

The present invention concerns a composition for the permanent shaping of human hair used both for the permanent waving of human hair with an excellent waving effect as well as for the straightening of curled hair.

It is generally known that permanent waving is carried out in two steps, the reductive splitting of the cysteine disulfide bonds in the hair by a reducing agent, and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycollates are customarily applied at a pH-value between 7 and 10, in particular 8.5 and 9.5.

Such compositions vary in their waving and/or straightening performance and, therefore, there is still need for further improvement.

The present invention starts from the task of providing a composition for the permanent shaping of human hair with excellent waving and straightening performance and at the same time giving or enhancing shine of hair. Hair waved or straightened with composition disclosed herein looks and feels natural upon touching by hand.

Accordingly, the first object of the present invention is a composition for permanent shaping and/or straightening hair based on at least one reducing agent, which further comprises at least one arylated silicone.

The compositions of the present invention comprises at least one arylated silicone at a concentration range of 0.01 to 5%, preferably 0.05 to 4% more preferably 0.1 to 3% and most preferably 0.1 to 2.5% by weight calculated to total composition prior to mixing with oxidizing lotion. Non-limiting suitable examples are phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane

In the preferred embodiment of the present invention, the arylated silicone comprises at least 2 phenyl groups, more preferably 3 and most preferably 5 phenyl groups in its molecule.

Particularly preferred arylated silicone is trimethyl pentaphenyl trisiloxane available from Dow Corning under the trade name DC PH-1555 HRI.

It should be noted that compositions of the present invention can also comprise more than one arylated silicone.

The permanent waving compositions according to the invention comprise at least one reducing compound. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycollate, 1.2-propyleneglycol monothioglycollate (see also WO-A 93/1791), 1-3-propanediol monothioglycollate or the isomer mixture resulting therefrom, 1.3-butanediol and 1.4-butanediol monothioglycollate and the isomer mixtures therefrom, polyethylene glycol, such as di-, tri- and tetraethyleneglycol monothioglycollates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 2.5 % to about 15 % by weight, calculated to total composition as free thioglycolic acid as reference substance.

The waving compositions containing reducing agents can, if necessary, comprise alkalizing agents. Their quantity is dependent on the reducing agent and the desired pH-value of the composition. Reducing agent compositions preferably contain about 0.1 % to about 5%, in particular about 0.5% to about 2.5% by weight thereof, calculated to the total composition. Alkalizing agents preferred within the scope of the invention are ammonia, ammonium carbamate, ammonia and/or ammonium(bi)carbonate, and alkanol amines with 1 to 4 carbon atoms such as monoethanolamine and triethanolamine. It is desirable to adjust the pH-value between about 6.5 and 10.5, preferably about 7 to 9.5.

The shaping compositions according to the invention are suited for use both for the permanent waving, i.e. curling of human hair and for the straightening, i.e. smoothing thereof.

The viscosity best suited for the permanent waving compositions according to the invention proved to be in the range of about 500 to 10,000 mPa.s, preferably about 1,000 to about 5,000 mPa.s, measured at 20°C in a Brookfield viscosimeter (no. 5 spindle), whereas the viscosity suited for the straightening compositions is preferably higher in a range up to about 50,000 mPa.s, preferably up to 30,000 mPa.s measured at 20°C in a Brookfield viscosimeter (no. 5 spindle).

The viscosity is adjusted by addition of the appropriate amounts of thickening agents known per se, such as cellulose derivatives. Thickening may as well be realised by formulating a composition in form of an emulsion with the use of C₁₀-C₂₂-fatty alcohols, in admixture with long mono alkyl chain quaternary ammonium surfactants.

The permanent waving compositions according to the present invention preferably comprise surfactants selected from anionic, nonionic, cationic and amphoteric ones. Their proportion ranges from about 0.05 % to about 10%, in particular from about 0.1 % to about 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise at least one cationic surfactant according to general formula 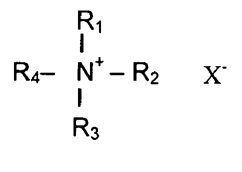 where R₁s a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, and X is chloride, bromide or methosulfate.

Concantration of at least one cationic surfactant is in the range from 0.05 % to 5 %, preferably 0.1 % to 2.5 % by weight, calculated to total composition.

Suitable long-chain quaternary ammonium compounds which can be used alone or in admixture are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

In a further preferred embodiment of the present invention, the compositions comprise at least one cationic polymer. Basically suitable are all cationic polymers listed under the generic name "Polyquaternium" and "Quaternium" in the CTFA International Cosmetic Ingredient Dictionary. Examples are Polyqauternium-4, Polyquaternium 6, Polyquaternium 7, Polyquaternium 10, Polyquaternium 11, Polyquaternium 16, Polyquaternium 22 and Polyquaternium 28, Polyquaternium 39, Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

The cationic polymers also include the quaternized products of graft polymers from organopolysiloxanes and polyethyl oxazolines described in EP-A 524 612 and EP-A 640 643.

Concantration of one or more cationic polymers is in the range from 0.05 % to 5 %, preferably 0.1 % to 2.5 % by weight, calculated to total composition.

Permanent shaping compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

Permanent shaping composition of the present invention can comprise further ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 2.5% by weight calculated to total composition.

Another preferred compound in the permanent shaping composition of the present invention is silicone compounds and especially aminated silicones such as amodimethicone available from for example Dow Corning under the brand names Dow Corning 949 Emulsion and Dow Corning 2-8194. Concentration of silicones, especially amodimethicone, is in the range of 0.05 to 2.5%, preferably 0.1 to 1% by weight calculated to total composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further preferred compound in the compositions of the present invention is ubichinone of the formula 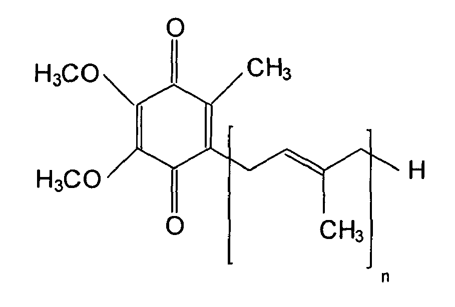 where n is a number between 1 and 10. Preferred ubichinones are the ones where n is a number between 6 and 10 and especially preferred is Ubichinone 50 where n is 10, also known as Coenzyme Q10.

Ubichinones have been used in cosmetic compositions. Such compositions have been disclosed for example in EP 751 762 B1 for hair conditioning compositions in combination with retinols, in DE 199 26 167 A1 and DE 199 26 168 A1 for hair styling compositions, DE 199 26 170 A1 for cleansing preparations and in DE 199 26 156 A1 for hair conditioning compositions. Furthermore, in EP 1232 741 A1 the use of ubichinones in hair colouring compositions is disclosed.

In none of the patent documents mentioned above, a permanent shaping composition for hair comprising ubichinone is neither disclosed nor mentioned.

Concentration of at least one ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Öle-Fette-Wachse", 117 (1991), pages 81 to 87.

Composition of the present invention is used in a process for permanent waving wherein hair is washed or shampooed first and wound on the curlers, subsequently a reducing agent comprising composition with at least one arylated silicone is applied onto hair and after 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers removed from hair. In cases where additional conditioning composition is required this may as well be applied.

In another process as described above the curlers are removed after rinsing off the reducing agent and before applying the oxidizing agent.

Further in another process, after rinsing off the reducing agent from hair, an intermediate treatment composition is applied onto hair and without rinsing off but after removing the excess amount of intermediate treatment with a towel, oxidizing composition is applied and at the end of the processing they are rinsed off from hair and curlers are removed from hair. It has further been found out that the use of at least one arylated silicone in the intermediate treatment composition in the same concentration range as disclosed above for the reducing composition improves the permanent shaping of hair as well in terms of curl appearance and natural look and feel of hair. Therefore, in another preferred form of the present invention, permanent shaping of hair is carried our with a process wherein hair is washed or shampooed first and subsequently a reducing agent comprising composition is applied onto hair and after 1 to 45 min, preferably 1 to 30 min of processing time, depending on the hair strength, rinsed off from hair with tap water and an intermediate treatment composition comprising at least one arylated silicone and magnesium sulfate at a concentration of 2 to 15% by weight is applied and without rinsing off an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off. In cases where additional conditioning composition is required this may as well be applied.

The intermediate treatment composition has a pH value between 2.5 to 6, preferably 3 to 5.5 and most preferably 3 to 5.

A straightening may also be carried out in a different process wherein hair is washed and/or shampooed and dried and reducing composition comprising at least one arylated silicone is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

In all of the above processes arylated silicone means all of the arylated silicone mentioned in the description. Here again the most preferred is pentaphenyl trimethyl trisiloxane.

The following examples are to illustrate the invention, but not to limit it.

### Example 1:

### Alkaline Permanent Wave for Normal Hair

| | | |
|---|---|---|
| Ammonium thioglycolate (60%) | 21.3 | (% by wt. |
| Ammonium hydrogen carbonate | 5.0 | |
| 1,3- butylene gylcol | 3.0 | |
| Trimethyl pentaphenyl trisiloxane | 0.2 | |
| PEG-40-Hydrogenated castor oil | 0.7 | |
| Polyquaternium-7 | 0.5 | |
| Perfume | 0.4 | |
| Coenzyme Q10 | 0.04 | |
| Ammonia, 25% | ad pH 8.3 | |
| Water | ad 100.0 | |

With this composition the hair was permanently waved for about 15 minutes, rinsed and neutralized for about 8 minutes with a customary 2.5% H₂O₂ composition. Homogeneous wave appearance was obtained. Exclusion of Trimethyl pentaphenyl trisiloxane resulted in less homogeneous perm appearance and especially less shiny hair.

### Example 2:

### Alkaline Permanent Wave for Damaged Hair

| | | |
|---|---|---|
| Ammonium thioglycollate (60%) | 15.0 | (% by wt.) |
| Ammonium hydrogen carbonate | 2.5 | |
| Ceteth-20 | 0.7 | |
| Trimethyl pentaphenyl trisiloxane | 0.1 | |
| 1,3- butylene gylcol | 0.5 | |
| Polyquaternium-6 | 1.5 | |
| Amodimethicone | 0.2 | |
| Coenzyme Q10 | 0.1 | |
| Oleic acid | 0.05 | |
| Perfume | 0.4 | |
| Ammonia, 25% | ad pH 8.0 | |
| Water | ad 100.0 | |

The permanent wave achieved with this composition was similar to the one obtained with the composition according to Example 1.

Exclusion of the Trimethyl pentaphenyl trisiloxane led to waves with substantially weaker contours and also significantly less shiny hair.

### Example 3:

### Alkaline Permanent Wave for Damaged Hair

| | | |
|---|---|---|
| Ammonium thioglycollate (60%) | 0.9 | (% by wt.) |
| Cystein hydrochloride | 5.7 | |
| Ammonium hydrogen carbonate | 1.5 | |
| Acetylcystein | 0.7 | |
| Cetrimonium chloride | 0.1 | |
| 1,3- butylene gylcol | 0.5 | |
| Trimethyl pentaphenyl trisiloxane | 0.5 | |
| Amodimethicone | 0.2 | |
| Coenzyme Q10 | 0.1 | |
| Oleic acid | 0.05 | |
| Perfume | 0.4 | |
| Ammonia, 25% | ad pH 9.8 | |
| Water | ad 100.0 | |

The permanent wave achieved with this composition was similar to the one obtained with the composition according to Example 1.

Exclusion of the Trimethyl pentaphenyl trisiloxane led to substantially weaker shine and waves did not look homogeneous..

### Example 4:

### Neutral Permanent Wave for Normal Hair

A permanent waving product consisting of two Compositions A and B, filled into a two-chamber packaging the chambers of which were kept separate until application, was prepared by destruction of the separating wall and applied onto human hair rolled onto curlers. The hair was rinsed after about fifteen minutes processing and neutralized for about five minutes with a 2.5 % H₂O₂ neutralizer composition, rinsed again, shampooed and dried.

An expressive, even, intensive permanent wave was obtained.

An identical treatment which had no pentaphenyl trimethyl trisiloxane showed a visibly inferior wave.

### Composition A:

| | | |
|---|---|---|
| Ammonium hydrogen carbonate | 4.5 | (g) |
| Polyquaternium -6 | 1.0 | |
| PEG-65-Hydrogenated castor oil | 0.8 | |
| Isopropyl alcohol | 1.5 | |
| Ethoxydiglycol | 2.0 | |
| Cocoamidopropyl betaine | 1.0 | |
| Trimethyl pentaphenyl trisiloxane | 0.2 | |
| Perfume | 0.3 | |
| Coenzyme Q10 | 0.05 | |
| Turbidifying agent | 0.5 | |
| Ammonia, 25% | ad pH 8.4 | |
| Water | ad 72.0 | |

### Composition B:

| | | |
|---|---|---|
| Ammonium thioglycollate, 70% | 18.0 | (g) |
| Thiolactic acid | 2.0 | |
| 2-Methyl-1.3-propanediol | 0.5 | |
| Ammonia, 25% | ad pH 5.5 | |
| Water | ad 28.0 | |

After admixture of both Compositions a ready-to-use product with a pH-value of 7.4 was obtained.

### Example 5:

### Neutral Permnent Wave for Dyed Hair

A permanent waving product filled into a two-chamber package was prepared in analogy to Example **4**

### Composition A:

| | | |
|---|---|---|
| Ammonium hydrogen carbonate | 3.5 | (g) |
| Polyquaternium -11 | 0.5 | |
| Ethanol | 0.5 | |
| 1-Methoxypropanol | 1.0 | |
| Cocoamidopropyl betaine | 1.0 | |
| Trimethyl pentaphenyl trisiloxane | 0.4 | |
| Coenzyme Q10 | 0.1 | |
| Oleic acid | 0.05 | |
| Perfume | 0.3 | |
| Turbidifying agent | 0.5 | |
| Ammonia, 25% | ad pH 8.3 | |
| Water | ad 72.0 | |

### Composition B:

| | | |
|---|---|---|
| Ammonium thioglycollate, 70% | 13.0 | (g) |
| Thiolactic acid | 0.5 | |
| 2-Methyl-1.3-propanediol | 1.5 | |
| Ammonia, 25% | ad pH 5.5 | |
| Water | ad 28.0 | |

A product with a pH-value of 7.4 was obtained by admixture of the Compositions immediately prior to application. After application onto dyed hair as described in Example 3, this mixture resulted in an expressive permanent wave, which had not effect whatever on the color gloss and color intensity.

### Example 6:

### Alkaline Permanent Waving Gel

| | | |
|---|---|---|
| Ammonium thioglycollate, 70% | 15.0 | (g) |
| Ammonium hydrogen carbonate | 4.5 | |
| PEG-40-Hydrogenated castor oil | 0.7 | |
| C₁₂-C₁₈-Fatty alcohol mixture | 3.5 | |
| Cetrimonium chloride | 2.0 | |
| Trimethyl pentaphenyl trisiloxane | 0.2 | |
| 2-Methyl-1.3-propanediol | 0.5 | |
| Polyquaternium -28 | 0.1 | |
| Coenzyme Q10 | 0.1 | |
| Perfume | 0.3 | |
| Ammonia, 25% | ad pH 8.0 | |
| Water | ad 100.0 | |

### Intermediate treatment composition

| | | |
|---|---|---|
| Asparagic acid | 0.25% | by weight |
| Glutamic acid | 0.50 | |
| Alanin DL | 0.25 | |
| Magnesium sulfate | 10.00 | |
| Trimethyl pentaphenyl trisiloxane | 0.1 | |
| Water | q.s. to 100 | |

The above composition had a pH of 4.10.

### Example 7:

### Straightening Composition

| | | |
|---|---|---|
| Thioglycolic acid | 8.0 | (% by wt.) |
| C₁₆-C₂₂-Fatty alcohol mixture | 3.5 | |
| Oleth-50 | 2.5 | |
| Laureth-23 | 1.5 | |
| Polyquaternium-2 | 0.8 | |
| Trimethyl pentaphenyl trisiloxane | 0.2 | |
| Ethanol | 5.0 | |
| Perfume | 0.6 | |
| Monoethanolamine | ad pH 9.3 | |
| Water | ad 100.0 | |

This composition constitutes an effecting smoothing composition for kinky hair.

## Claims

1. Composition for the permanent shaping of human hair, comprising at least one reducing agent, **characterized in that** it comprises at least one arylated silicone.

2. Composition according to any of the preceding claims **characterised in that** at least one arylated silicone is selected from phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane.

3. Composition according to any of the preceding claims **characterised in that** at least one arylated silicone is trimethyl pentaphenyl trisiloxane.

4. Composition according to any of the preceding claims **characterised in that** it comprises at least 2 arylated silicones.

5. Composition according to any of the preceding claims **characterised in that** it comprises arylated silicone at a concentration of 0.01 to 5% by weight calculated to total composition.

6. Composition according to any of the preceding claims, **characterized in that** the reducing agents are selected from thioglycolic acid, thiolactic acid and their salts, cystein and its hydrochloride salt, acetylcystein.

7. Composition according to one or more of claims 1 to 3, **characterized in that** it has a Brookfield viscosity of 500 to 10,000 mPa.s at 20°C.

8. Composition according to any of the preceding claims **characterized in that** it comprises at least one surfactant selected from anionic, nonionic, cationic and amphoteric ones at a concentration of 0.05 to 10% by weight, calculated to total composition.

9. Composition according to any of the preceding claims **characterized in that** it comprises at least one cationic polymer at a concentration of 0.05 to 5% by weight, calculated to total composition.

10. Composition according to any of the preceding claims **characterized in that** it comprises at least one organic solvent at a concentration of 0.1 to 10% by weight, calculated to total composition.

11. Composition according to any of the preceding claims **characterized in that** it comprises at least one aminated silicone at a concentration of 0.05 to 2.5% by weight, calculated to total composition.

12. Composition according to any of the preceding claims **characterized in that** it comprises at least one fatty acid with 10 to 22 C atoms at a concentration of 0.01 to 2.5% by weight, calculated to total composition.

13. Composition according to any of the preceding claims **characterized in that** it comprises at least one ubichinone of the formula 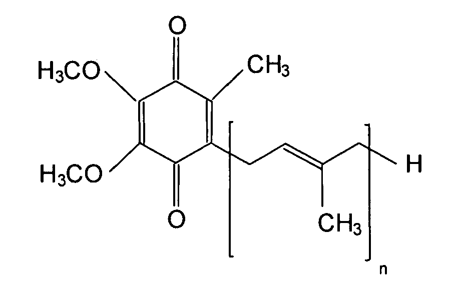 where n is a number between 1 and 10 at a concentration of 0.0001 - 1 % by weight, calculated to total composition.

14. Use of composition according to any of the proceeding claims for permanent shaping hair.

15. Process for permanent waving hair **characterized in that** hair is washed or shampooed first and wound on curlers and subsequently a composition according to claims 1 to 14 is applied onto hair and at the end of the 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair.

16. Process according to claim 16 **characterized in that** after rinsing off the reducing agent from hair, an intermediate treatment composition optionally comprising at least one arylated silicone is applied onto hair and without rinsing off and after removal of the excess amount with towel, an oxidizing composition is applied and at the end of the processing they are rinsed off from hair.

17. Process for straightening hair **characterized in that** hair is washed and/or shampooed and dried and reducing composition according to claims 1 to 14 is applied onto dry hair and processed for 5 to 60 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.
